(19) Europäisches Patentamt
European Patent Office
Office européen des brevets

(11) Veröffentlichungsnummer: **0 169 439**
**B1**

# (12) EUROPÄISCHE PATENTSCHRIFT

(45) Veröffentlichungstag der Patentschrift: **16.08.89**

(51) Int. Cl.⁴: **C 07 D 249/08**, C 07 D 303/48, C 07 D 405/04

(21) Anmeldenummer: **85108573.8**

(22) Anmeldetag: **10.07.85**

## (54) Verfahren und Zwischenprodukte zur Synthese von diastereomeren Triazolyl-O,N-acetalen.

(30) Priorität: **20.07.84 DE 3426906**

(43) Veröffentlichungstag der Anmeldung: **29.01.86 Patentblatt 86/5**

(45) Bekanntmachung des Hinweises auf die Patenterteilung: **16.08.89 Patentblatt 89/33**

(84) Benannte Vertragsstaaten: **AT BE CH DE FR GB IT LI NL**

(56) Entgegenhaltungen:
**GB-A- 2 102 796**

**TETRAHEDRON LETTERS, Band 26, Nr. 36, 1985, Seiten 4337-4344, Pergamon Press Ltd., GB; J. GASTEIGER et al.: "Nucleophilic substitution at a saturated carbon atom with retention of configuration: The reaction of trans-2-halo-3-tert-butyloxiranes with phenolates"**

(73) Patentinhaber: **BAYER AG, D-5090 Leverkusen 1 Bayerwerk (DE)**

(72) Erfinder: **Gasteiger, Johann, Dr., Alfred-Kubin-Weg 36, D-8000 München 71 (DE)**
Erfinder: **Kaufmann, Karlheinz, Dr., Walpurgisstrasse 14, D-8000 München 80 (DE)**
Erfinder: **Mengel, Rudolf, Dr., Im Herzenacker 32, D-6535 Gau-Algesheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 169 439 B1

**Beschreibung**

Die Erfindung beschreibt ein neues Verfahren zur gezielten Synthese der beiden diastereomeren Formen von Triazolyl-O,N-acetalen der Formel

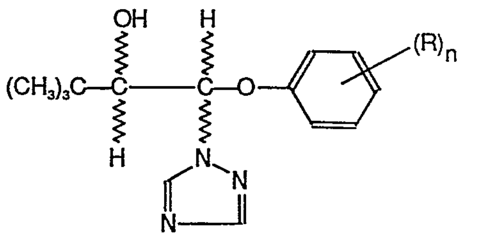

(I)

in der R und n die im Text angegebene Bedeutung haben, sowie neue trans-substituierte Oxirane und deren Verwendung zur Synthese von Verbindungen der Formel (I).

Es ist bekannt, dass die fungizid wirksamen Triazolyl-O,N-acetale der Formel (I) in zwei unterschiedlichen diastereomeren Formen (threo und erythro) vorliegen können. Das 1-(4-Chlorphenoxy)-3,3-dimethyl-1-(1,2,4-triazol-1-yl)-butanol-2 ist unter dem Namen Triadimenol als Fungizid im Handel [DE-A 2 324 010].

Beim biologischen Abbau des ebenfalls fungizid wirksamen Triadimefon [1-(4-Chlorphenoxy)-3,3-dimethyl-1-(1,2,4-triazol-1-yl)-butanon-2] [DE-A 2 201 063] wird ein Metabolismus vorgeschlagen, bei dem unter Reduktion der Ketogruppe vorwiegend eine der beiden diastereomeren Formen des Triadimenols entsteht.

Es wird diskutiert, dass diese Metaboliten mit der fungitoxischen Wirkung des Triadimefons im Zusammenhang stehen [Pestic. Sci. 1984 71–77; Zeitschr. f. Pflanzenkr., 1982 309].

Aus der DE-A 2 743 767 ist ein Verfahren zur Reduktion von Triadimefon und Verbindungen ähnlicher Struktur bekannt, bei dem vorwiegend eines der beiden Diastereomeren gebildet wird. Dieses Verfahren hat den Nachteil, dass das Hauptprodukt erst durch Reinigungsverfahren vom Nebenprodukt getrennt werden muss. Zudem ist so nur eine der beiden diastereomeren Formen unter vertretbarem Aufwand zugänglich.

Es wurde nun gefunden, dass sich die diastereomeren Formen (A) und (B) von Triazolyl-O,N-acetalen der Formel

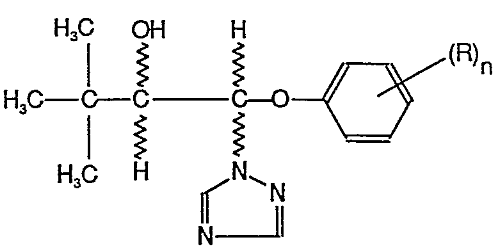

(I)

in welcher

R für gegebenenfalls durch Halogen, Alkyl mit 1 bis 4 Kohlenstoffatomen und/oder Halogenalkyl mit 1 oder 2 Kohlenstoffatomen und 1 bis 5 Halogenatomen substituiertes Phenyl, für gegebenenfalls durch Halogen, Alkyl mit 1 bis 4 Kohlenstoffatomen und/oder Halogenalkyl mit 1 oder 2 Kohlenstoffatomen und 1 bis 5 Halogenatomen substituiertes Phenoxy, für gegebenenfalls durch Halogen substituiertes Alkyl mit 1 bis 4 Kohlenstoffatomen, für gegebenenfalls durch Halogen substituiertes Alkoxy mit 1 bis 4 Kohlenstoffatomen, für gegebenenfalls durch Halogen substituiertes Aklylthio mit 1 bis 4 Kohlenstoffatomen, für Alkylcarbonyl mit 1 bis 4 Kohlenstoffatomen in der Alkylgruppe, Nitro, Fluor, Chlor, Brom und Iod steht und

n für die Zahlen 1, 2 oder 3 steht, nach einem neuen Verfahren dadurch herstellen lassen, dass man Oxirane der Formel

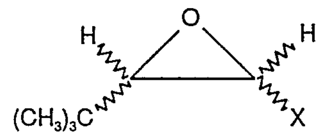

(II)

in welcher

X für Chlor oder Brom steht,

entweder

a) mit Phenolen der Formel

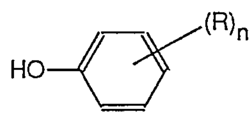

(III)

in welcher

R und n die oben angegebene Bedeutung haben, gegebenenfalls in Gegenwart einer Base sowie gegebenenfalls in Gegenwart eines Verdünnungsmittels bei Temperaturen zwischen −10 °C und +100 °C umsetzt und die dabei anfallenden Oxiran-Derivate der Formel

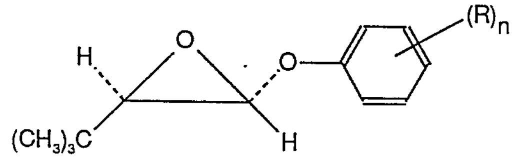

(IV)

in welcher

R und n die oben angegebene Bedeutung haben, in einem zweiten Reaktionsschritt mit 1,2,4-Triazol in Gegenwart einer Base, oder dem Natrium- oder Kalium-Salz des 1,2,4-Triazols, gegebenenfalls in Gegenwart eines Verdünnungsmittels bei Temperaturen zwischen 50 °C und 120 °C zu threo-Isomeren der Formel

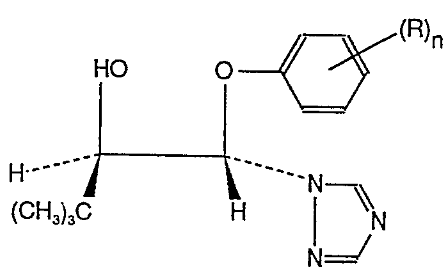

(IA)

in welcher
R und n die oben angegebene Bedeutung haben, umsetzt, oder
b) mit 1,2,4-Triazol in Gegenwart einer Base, oder dem Natrium- oder Kalium-Salz des 1,2,4-Triazols, gegebenenfalls in Gegenwart eines Verdünnungsmittels bei Temperaturen zwischen 0°C und 60°C umsetzt und das dabei anfallende Oxiran-Derivat der Formel

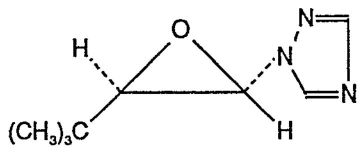

(V)

in einem zweiten Reaktionsschritt mit Phenolen der Formel

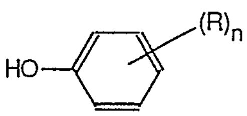

(III)

in welcher
R und n die oben angegebene Bedeutung haben, gegebenenfalls in Gegenwart einer Base sowie gegebenenfalls in Gegenwart eines Verdünnungsmittels bei Temperaturen zwischen 50°C und 120°C zu erythro-Isomeren der Formel

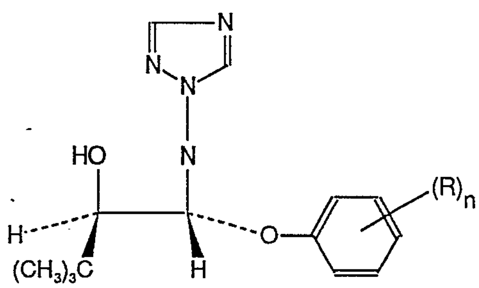

(IB)

in welcher
R und n die oben angegebene Bedeutung haben, umsetzt.

Überraschenderweise reagieren trans-3-tert.-Butyl-2-halogen-oxirane der Formel

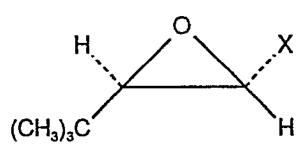

(II-a)

bei der Durchführung des erfindungsgemässen Verfahrens mit Nucleophilen, wie zum Beispiel Natriumphenolaten oder Natrium-1,2,4-triazolid, unter Beibehaltung des Oxiranringes und unter Konfigurationserhalt am Dreiring zu den entsprechenden trans-substituierten Oxiran-Derivaten der Formeln (IV) bzw. (V).

Auch bei Verwendung von cis-trans-Gemischen von Oxiranen der Formel (II) bilden sich überwiegend die trans-substituierten Oxiran-Derivate der Formeln (IV) bzw. (V).

Bei der anschliessenden Umsetzung dieser Oxiran-Derivate der Formeln (IV) bzw. (V) mit einem zweiten Nucleophil reagiert das Oxiran-System der Verbindungen der Formeln (IV) bzw. (V) unter Ringöffnung, wobei das eintretende Nucleophil an das Kohlenstoffatom gebunden wird, das bereits das erste Nucleophil trägt. Weil der Eintritt des zweiten Nucleophils ausserdem von der dem Oxiran-Sauerstoff abgewandten Seite aus stattfindet, ist die Stereochemie des jeweils entstehenden Produktes der Formel (I) eindeutig difiniert.

Die bei der Durchführung des erfindungsgemässen Verfahrens als Ausgangsprodukte benötigten Oxirane sind durch die Formel (II) definiert.

Die Oxirane der Formel (II) sind bekannt oder lassen sich nach Verfahren, die aus der Literatur bekannt sind, aus Pinakolon oder Pivalaldehyd herstellen. So erhält man Oxirane der Formel (II), indem man Pinakolon durch Halogenierung in ein α,α-Dihalogenketon der Formel

$(CH_3)_3C\text{-}CO\text{-}CHX_2$ (VI)

in welcher
X die oben angegebene Bedeutung hat, überführt, welches dann bei der anschliessenden Reduktion unter alkalischen Bedingungen cyclisiert wird.

Ausserdem lassen sich Oxirane der Formel (II) synthetisieren, indem man an Pivalaldehyd ein Dihalogencarbenoid addiert und die dabei entstehenden Alkohole der Formel

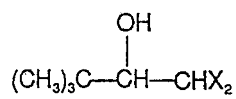

(VII)

in welcher
X die oben angegebene Bedeutung hat, unter basischen Bedingungen cyclisiert. Dieses Verfahren kann sowohl einstufig als auch zweistufig durchgeführt werden.

Die bei der Durchführung des erfindungsgemässen Verfahrens weiterhin als Ausgangsstoffe benötigten Phenole sind durch die Formel (III) definiert.

Besonders bevorzugt verwendbar sind Phenole der Formel (III), in denen
R für gegebenenfalls durch Fluor, Chlor, Brom, Methyl, Ethyl, n-Propyl, Isopropyl, n-Butyl, sek.-Butyl, iso-Butyl, tert.-Butyl, Trifluormethyl, Trichlormethyl, Dichlorfluormethyl und/oder Difluorchlormethyl substituiertes Phenyl, für gegebenenfalls durch Fluor, Chlor, Brom, Methyl, Ethyl, n-Propyl, Isopropyl, n-Butyl, sek.-Butyl, iso-Butyl, tert.-Butyl, Trifluormethyl, Trichlormethyl, Dichlorfluormethyl und/oder Difluorchlormethyl substituiertes Phenoxy, für Methyl, Ethyl, n-Propyl, Isopropyl, n-Butyl, iso-Butyl, sek.-Butyl, tert.-Butyl, Trifluormethyl, Trichlormethyl, Dichlorfluormethyl, Difluorchlormethyl, Methoxy, Ethoxy, Trifluormethoxy, Methylthio, Ethylthio, Trifluormethylthio, Methylcarbonyl, Ethylcarbonyl, Nitro, Fluor, Chlor und Brom steht und

n für 1, 2 oder 3 steht. Als Beispiele für Phenole der Formel (III) seien die in der folgenden Tabelle 1 aufgeführten Stoffe genannt.

Tabelle 1

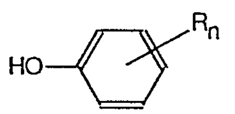

(III)

| $R_n$ | $R_n$ |
|---|---|
| 4-Cl | 2-$CH_3$—CO— |
| 4— 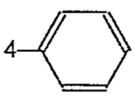 | 4-$CF_3$O— |
| 2,4-Cl | 4-(4-$CF_3$— 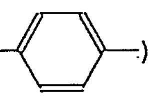 —) |
| 4-$CF_3$ | 4-(4-Cl— 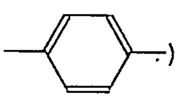 —) |
| 4-$NO_2$ | |
| 2,4,6-$Cl_3$ | 4-(2,4-$Cl_2$— 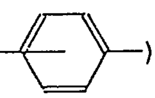 —) |
| 4-F | 4-(4-$CH_3$— 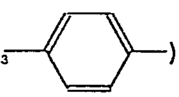 —) |
| 3-Cl | |
| 3,5-$Cl_2$ | 4— 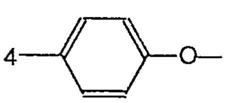 —O— |

Die Phenole der Formel (III) sind bekannt oder lassen sich nach bekannten Methoden herstellen.

Die bei der Durchführung des erfindungsgemässen Verfahrens nach der Variante (a) entstehenden Oxiran-Derivate der Formel (IV) sind neu. In dieser Formel haben R und n vorzugsweise diejenigen Bedeutungen, die bereits im Zusammenhang mit der Beschreibung der Phenole der Formel (III) für den Rest R und den Index n als besonders bevorzugt genannt wurden.

Als Beispiele für Oxiran-Derivate der Formel (IV) seien die in der folgenden Tabelle 2 aufgeführten Stoffe genannt.

Tabelle 2

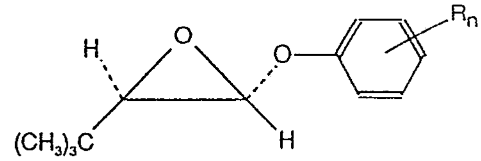

(IV)

| $R_n$ | $R_n$ |
|---|---|
| 4-Cl | 2-$CH_3$—CO— |
| 4— 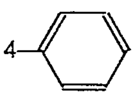 | 4-$CF_3$O— |
| 2,4-$Cl_2$ | 4-(4-$CF_3$— 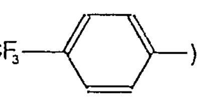 —) |
| 4-$CF_3$ | 4-(4-Cl— 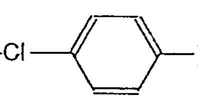 —) |
| 2,4,6-$Cl_3$ | 4-(4-$CH_3$— 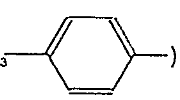 —) |
| 4-F | 4— 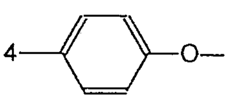 —O— |
| 3-Cl | |
| 3,5-$Cl_2$ | 4-(Cl— 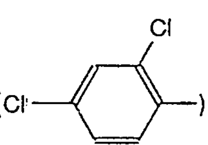 —) |

Das bei der Durchführung des erfindungsgemässen Verfahrens nach der Variante (b) entstehende Oxiran-Derivat der Formel (V) ist ebenfalls neu.

Die nach dem erfindungsgemässen Verfahren herstellbaren diastereomeren Formen (A) und (B) von Triazolyl-O,N-acetalen sind durch die Formeln (IA) bzw. (IB) definiert. In diesen Formeln haben R und n jeweils vorzugsweise diejenigen Bedeutungen, die bereits im Zusammenhang mit der Beschreibung der Phenole der Formel (III) für den Rest R und den Index n als besonders bevorzugt genannt wurden.

Als Beispiele für Diastereomere der Formeln (IA) und (IB) seien die in der folgenden Tabelle 3 aufgeführten Stoffe genannt.

Tabelle 3

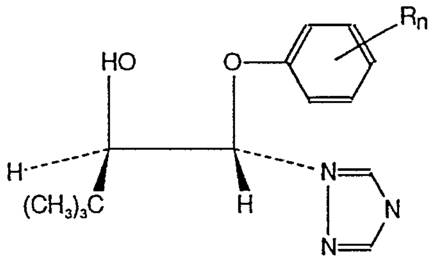

(IA) threo

und

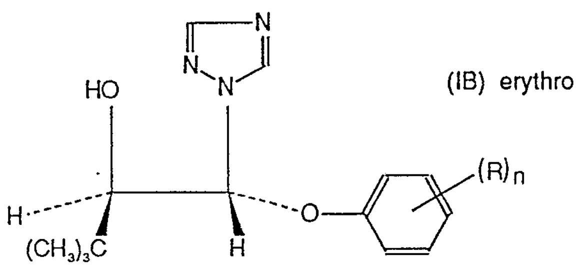

(IB) erythro

| $R_n$ | $R_n$ |
|---|---|
| 4-Cl | 3,5-$Cl_2$ |
| 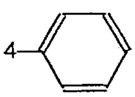 | 2-$CH_3$—CO— |
| 2,4-$Cl_2$ | 4-$CF_3O$— |
| 4-$CF_3$ | 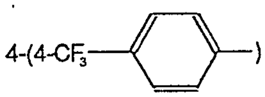 |
| 4-$NO_2$ | 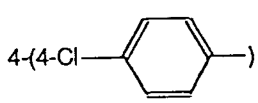 |
| 2,4,6-$Cl_3$ | 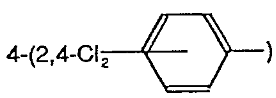 |
| 4-F | 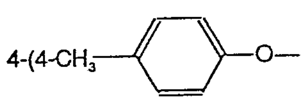 |
| 3-Cl | 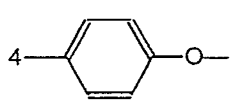 |

Bei der Durchführung des erfindungsgemässen Verfahrens nach der Variante (a) werden die Phenole der Formel (III) entweder in Gegenwart von Basen oder aber in Form von Phenolaten eingesetzt. Als Basen kommen hierbei vorzugsweise Alkalihydroxide, wie Natriumhydroxid oder Kaliumhydroxid, ferner Hydride, wie Natriumhydrid, und ausserdem Alkoholate, wie Kalium-tert.-butylat, in Betracht. Als Phenolate kommen vorzugsweise Alkaliphenolate, wie Natrium- oder Kaliumphenolate, in Frage.

Als Verdünnungsmittel können bei der Durchführung des erfindungsgemässen Verfahrens nach der Variante (a) in der ersten Stufe alle für derartige Reaktionen üblichen inerten, organischen Solventien eingesetzt werden. Vorzugsweise in Betracht kommen Nitrile, wie Acetonitril, ferner aromatische Kohlenwasserstoffe, wie Benzol, Toluol und Xylol, weiterhin Ether, wie Diethylether und Tetrahydrofuran, und ausserdem polare, aprotische Lösungsmittel, wie Dimenthylformamid und Dimethylsulfoxid.

Bei der Durchführung des erfindungsgemässen Verfahrens nach der Variante (a) wird in der zweiten Stufe das Triazol entweder in Gegenwart von Basen, wie Kalium- oder Natrium-hydroxid, oder aber auch in Form des Triazol-Natrium- oder Triazol-Kalium-Salzes eingesetzt.

Als Verdünnungsmittel kommen bei der Durchführung der zweiten Stufe des erfindungsgemässen Verfahrens in der Variante (a) vorzugsweise Alkohole, wie Methanol oder Ethanol, in Frage.

Die Reaktionstemperaturen können bei der Durchführung der Variante (a) des erfindungsgemässen Verfahrens innerhalb eines bestimmten Bereiches variiert werden. Bei der Durchführung der ersten Stufe arbeitet man bei Temperaturen zwischen −10°C und +100°C, vorzugsweise zwischen 0°C und 50°C. Bei der Durchführung der zweiten Stufe arbeitet man bei Temperaturen zwischen 50°C und 120°C, vorzugsweise zwischen 60°C und 100°C.

Bei der Durchführung des erfindungsgemässen Verfahrens nach der Variante (b) wird das Triazol entweder in Gegenwart von Basen, wie Kalium- oder Natriumhydroxid, oder aber in Form des Triazol-Natrium- oder Triazol-Kalium-Salzes eingesetzt.

Als Verdünnungsmittel können bei der Durchführung des erfindungsgemässen Verfahrens (b) alle für derartige Reaktionen üblichen inerten, organischen Solventien eingesetzt werden. Vorzugsweise in Betracht kommen alle diejenigen Lösungsmittel, die auch in Zusammenhang mit der Durchführung der ersten Stufe der erfindungsgemässen Umsetzung nach der Variante (a) vorzugsweise erwähnt wurden.

Bei der Durchführung des erfindungsgemässen Verfahrens nach der Variante (b) werden die Phenole der Formel (III) in der zweiten Stufe entweder in Gegenwart von Basen oder aber in Form von Phenolaten eingesetzt. Als Basen bzw. Phenolate kommen vorzugsweise diejenigen in Betracht, die bereits im Zusammenhang mit der Variante (a) vorzugsweise genannt wurden.

Als Verdünnungsmittel kommen bei der Durchführung der zweiten Stufe des erfindungsgemässen Verfahrens nach der Variante (b) wiederum vorzugsweise Alkohole, wie Methanol oder Ethanol, in Frage.

Auch bei der Durchführung der Variante (b) des erfindungsgemässen Verfahrens können die Reaktionstemperaturen innerhalb eines bestimmten Bereiches variiert werden. Bei der Durchführung der ersten Stufe arbeitet man bei Temperaturen zwischen 0°C und 60°C, vorzugsweise zwischen 10°C und 50°C. Bei der Durchführung der zweiten Stufe arbeitet man bei Temperaturen zwischen 50°C und 120°C, vorzugsweise zwischen 60°C und 100°C.

Die Aufarbeitung und die Isolierung der Reaktionsprodukte erfolgt sowohl bei der Durchführung der Variante (a) als auch der Variante (b) des erfindungsgemässen Verfahrens nach üblichen Methoden.

Die nach dem erfindungsgemässen Verfahren herstellbaren Stoffe besitzen sehr gute fungizide Eigenschaften.

Das erfindungsgemässe Verfahren wird durch die folgenden Beispiele veranschaulicht.

Beispiel 1

$(CH_3)_3C\text{-}CO\text{-}CHCl_2$ (VI-1)

In eine Mischung von 40 g Pinakolon und 10 ml Wasser wird bei 0°C bis zur Sättigung Chlor eingeleitet. Die dabei entstehende Dichlorverbindung kristallisiert spontan aus und wird aus Petrolether (60–90°C) umkristallisiert.

Ausbeute: 27,2 g (45% der Theorie) 1,1-Dichlor-3,3-dimethyl-butanon-2 als farblose Nadeln vom Schmelzpunkt 51°C.

Beispiel 2

$(CH_3)_3C\text{-}CO\text{-}CHBr_2$ (VI-2)

In eine Mischung von 100 g Pinakolon mit 300 ml Diethylether und 180 ml Dioxan werden bei 35–40°C unter Rühren 35,00 g Brom getropft. Gegen Ende der Reaktion verfärbt sich die Lösung. Sie wird mit $NaHSO_3$/Wasser bis zum Verschwinden der Bromfarbe geschüttelt, mit Wasser gewaschen und am Rotationsverdampfer eingeengt. Das dabei ausfallende, schwach orange Punkt wird aus n-Hexan umkristallisiert.

Ausbeute: 206 g (81% der Theorie) 1,1-Dibrom-3,3-dimethyl-butanon-2 vom Schmelzpunkt 75°C.

Beispiel 3

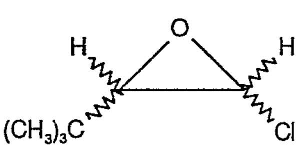

(II–1)

Eine Lösung von 67,6 g 1,1-Dichlor-3,3-dimethyl-butanon-2 in 160 ml Diethylether wird in eine Lösung von 28,0 g Natriumhydroxid in 120 ml Wasser und 50 ml Methanol getropft. Unter Kühlung auf −10°C werden unter Rühren portionsweise 10 g $NaBH_4$ eingetragen. Nach Abklingen der Reaktion wird noch weitere 24 h bei Raumtemperatur nachgerührt. Die organische Phase wird abgetrennt, die wässrige Phase zweimal mit Ether ausgeschüttelt, die vereinigten organischen Phasen werden getrocknet und bei 30°C im Vakuum über eine 1 m lange Vigreuxkolonne vorsichtig eingeengt.

Die Rohlösung des Oxirans in Ether ist 82%ig und enthält Verunreinigungen von maximal 1%.

Ausbeute: 62,9 g Lösung = 51,6 g (3-tert.-Butyl-2-chloroxiran (96% der Theorie).

Durch Destillation der etherischen Lösung wird eine Hauptfraktion isoliert.

Ausbeute: 48,7 g (90% der Theorie) 3-tert.-Butyl-2-chlor-oxiran als farblose Flüssigkeit mit $Kp_{13}$ = 31°C. Das Isomerenverhältnis cis:trans wird NMR-spektroskopisch bestimmt: cis:trans = 12:88

Beispiel 4

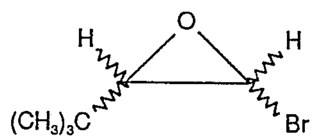

(II–2)

Nach der im Beispiel 3 beschriebenen Methode erhält man aus 51,6 g 1,1-Dibrom-3,3-dimethyl-butanon-2 als Produkt das 2-Brom-3-tert.-butyloxiran.

Ausbeute: 25,8 g (72% der Theorie) 2-Brom-3-tert.-butyloxiran als 1:9 Gemisch der cis/trans-Isomere

Beispiel 5

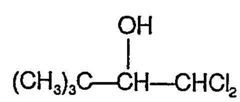

(VII–1)

Bei −80°C werden 0,20 mol Butyllithium (1,6 n in Hexan) zu einer Mischung aus 17,00 g Methylenchlorid in 300 ml Tetrahydrofuran, Ether, Hexan (4:1:1) getropft. Es wird 30 Minuten bei der gleichen Temperatur nachgerührt und dann eine Lösung von 17,2 g Pivalaldehyd in 100 ml Ether zugetropft. Die Reaktionsmischung wird für 12 Stunden in einem Dewar-Gefäss belassen, wo sie sich langsam erwärmt. Anschliessend wird am Rotationsverdampfer eingeengt und der zähflüssige Rückstand in je 50 ml Ether und Wasser aufgenommen. Die wässrige Phase wird noch zweimal mit Ether extrahiert, die vereinigten Phasen über $Na_2SO_4$ getrocknet, das Lösungsmittel abdestilliert und dann aus Pentan umkristallisiert.

Ausbeute: 20,6 g (67% der Theorie) 1,1-Dichlor-3,3-dimethyl-butanol-2 als farblose Nadeln vom Schmelzpunkt 49°C.

Beispiel 6

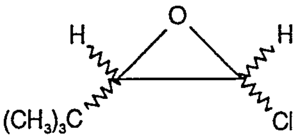

(II–1)

Zu 17,10 g 1,1-Dichlor-3,3-dimethyl-butanol-2 werden unter starkem Rühren bei 0°C 37,0 ml 3-n wässrige Natronlauge zugetropft. Nach 1 Stunde wird die Eiskühlung entfernt und weitere 14 Stunden bei Raumtemperatur gerührt. Dann wird die organische Phase abgetrennt, mit Wasser gewaschen, über einem Molekularsieb (4Å) getrocknet und im Wasserstrahlvakuum destilliert.

Ausbeute: 11,4 g (85% der Theorie) 2-Chlor-3-tert.-butyloxiran als Flüssigkeit mit $Kp_{13}$:35°C. Das cis/trans-Isomerenverhältnis beträgt: cis:trans = 12:88

Beispiel 7

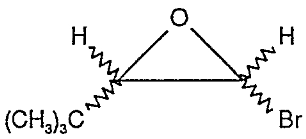

(II–2)

Zu 22,4 g Diisopropylamin in 400 ml Tetrahydrofuran, 100 ml Diethylether und 100 ml Hexan tropft man bei −10°C 0,20 mol Butyllithium (1,6 n in Hexan) und lässt die Lösung 1 Stunde bei Raumtemperatur ausreagieren.

Anschliessend wird auf −80°C abgekühlt und im Laufe von 45 Minuten eine Lösung von 17,00 g Dibrommethan in 40 ml Diethylether zugetropft. Nach weiteren 30 Minuten werden 17,2 g Pivalaldehyd gelöst in 70 ml Diethylether innerhalb von 1 Stunde vorsichtig zugetropft. Es wird 15 Stunden nachgerührt, wobei sich die Reaktionslösung langsam auf Raumtemperatur erwärmt. Nach dem Einengen am Rotationsverdampfer wird die Suspension in je 50 ml Wasser und Diethylether aufgenommen, die organische Phase mit Wasser gewaschen, über $Na_2SO_4$ getrocknet, am Rotationsverdampfer eingeengt und im Wasserstrahlvakuum destilliert.

Ausbeute: 17,55 g (40% der Theorie) 2-Brom-3-tert.-butyloxiran als Flüssigkeit mit $Kp_{11}$ = 42°C.

Das Isomerenverhältnis cis:trans wird NMR-spektroskopisch bestimmt: cis/trans = 1,5/98,5.

Beispiel 8

Synthese der beiden diastereomeren Formen von 1-(4-Chlorphenoxy)-3,3-dimethyl-1-(1,2,4-triazol-1-yl)-butanol-2

a) trans-2-tert.-butyl-3-(4-chlorphenoxy)-oxiran

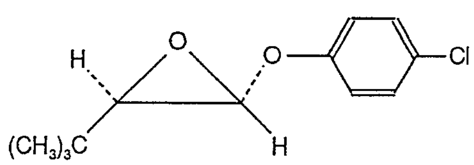

(IV–1)

Zu 10,0 g eines cis/trans-Isomerengemisches von 2-Brom-3-tert.-butyloxiran (cis/trans = 1/9) in 5 ml p-Chlorphenolat, in 15 ml Acetonitril suspendiert getropft. Die Lösung wird noch 50 Stunden bei Raumtemperatur gerührt. Dabei verfärbt sich die Lösung schwach bräunlich und es bildet sich ein weisser Niederschlag. Die Suspension wird eingeengt und in je 20 ml Diethylether und Wasser aufgenommen. Die organische Phase wird mit 3-n wässriger Natronlauge und Wasser gewaschen, über $Na_2SO_4$ getrocknet, eingeengt und im Hochvakuum destilliert.

Ausbeute: 8,85 g (71% der Theorie) trans-2-tert.-Butyl-3-(4-Chlorphenoxy)-oxiran mit $Kp_{0,01}$: 55°C

b) trans-3-tert.-Butyl-2-(1,2,4-triazol-1-yl)-oxiran

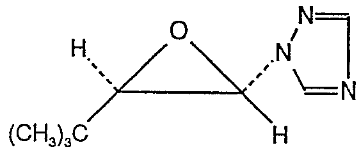

(V–1)

Zu einer Suspension von 5,92 g (65 mmol) Natrium-1,2,4-triazol in 70 ml Acetonitril, werden bei Raumtemperatur 10,62 g (59 mmol) 2-Brom-3-tert.-butyloxiran (cis:trans = 1,5:98,5) und 1,50 15-Krone-5 gegeben. Die Suspension wird 140 Stunden bei 30°C gerührt. Anschliessend wird das Lösungsmittel am Rotationsverdampfer entfernt, der Rückstand in Ether aufgenommen und eingeengt. Das schwach gelbliche Rohprodukt (5,0 g; 50% der Theorie) wird durch Destillation im Hochvakuum gereinigt.

Ausbeute: 3,85 g (39% der Theorie) trans-3-tert.-Butyl-2-(1,2,4-triazol-1-yl)oxiran als farblose Flüssigkeit mit Kp: 34°C bei $6 \times 10^{-3}$m bar.

c) threo-1-(4-Chlorphenoxy)-3,3-dimethyl-1-(1,2,4-triazol-1-yl)-butanol-2

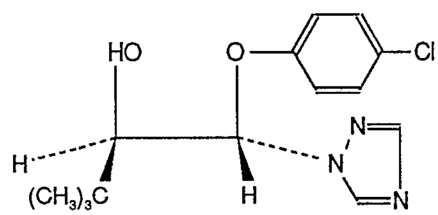

(IA–1)

1,13 g trans-2-tert.-Butyl-(4-chlorphenoxy)-oxiran werden zusammen mit 0,68 g Natriumtriazolid in 10 ml Methanol gelöst und 22 Stunden unter Rückfluss gerührt. Anschliessend wird das Lösungsmittel abdestilliert, der Rückstand in je 25 ml Dichlormethan und Wasser aufgenommen, die organische Phase abgetrennt, mit Wasser nachgewaschen, über $Na_2SO_4$ getrocknet, erneut eingeengt und an Kieselgel mit Petrolether/Essigester (2:8) getrennt.

Ausbeute: 0,89 g (60% der Theorie) threo-1-(4-Chlorphenoxy)-3,3-dimethyl-1-(1,2,4-triazol-1-yl)-butanol-2 als farblose Kristalle vom Schmelzpunkt 138–139°C.

und als Nebenprodukte

0,09 g (6% der Theorie) threo-1-(4-Chlorphen-

oxy)-3,3-dimethyl-1-(1,2,4-triazol-2-yl)-butanol-2 vom Schmelzpunkt 216–218 °C.

0,08 g (9,6% der Theorie) 3,3-Dimethyl-2-(1,2,4-triazol-1-yl)-butanol als schwach gelbliche Flüssigkeit.

d) erythro-1-(4-Chlorphenoxy)-3,3-dimethyl-1-(1,2,4-triazol-1-yl)-butanol-2

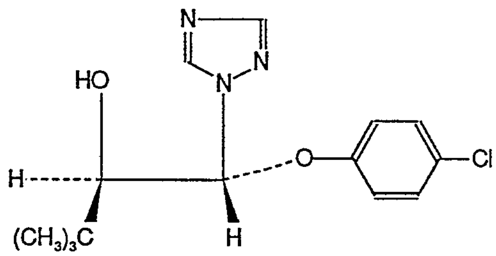

(IB–1)

0,84 g trans-2-tert.-Butyl-3-(1,2,4-triazol-1-yl)-oxiran werden mit 2,25 g Natrium-p-chlorphenolat in 10 ml Methanol für 120 Stunden unter Rückfluss erhitzt. Das Lösungsmittel wird entfernt, der Rückstand in Essigester aufgenommen und mit 3 n wässriger NaOH und Wasser gewaschen. Die organische Phase wird mit $Na_2SO_4$ getrocknet, eingeengt und an Kieselgel mit Petrolether/Essigester (2:8) chromatographiert.

Ausbeute: 0,25 g (17% der Theorie) erythro-1-(4-Chlorphenoxy)-3,3-dimethyl-1-(1,2,4-triazol-1-yl)-butanol-2 als farblose Kristalle vom Schmelzpunkt 132 °C.

**Patentansprüche**

1. Verfahren zur Synthese der diastereomeren Formen (A) und (B) von Triazolyl-O,N-acetalen der Formel

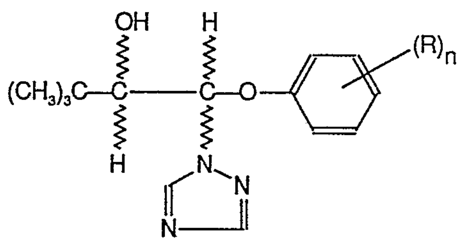

(I)

in welcher

R für gegebenenfalls durch Halogen, Alkyl mit 1 bis 4 Kohlenstoffatomen und/oder Halogenalkyl mit 1 oder 2 Kohlenstoffatomen und 1 bis 5 Halogenatomen substituiertes Phenyl, für gegebenenfalls durch Halogen, Alkyl mit 1 bis 4 Kohlenstoffatomen und/oder Halogenalkyl mit 1 oder 2 Kohlenstoffatomen und 1 bis 5 Halogenatomen substituiertes Phenoxy, für gegebenenfalls durch Halogen substituiertes Alkyl mit 1 bis 4 Kohlenstoffatomen, für gegebenenfalls durch Halogen substituiertes Alkoxy mit 1 bis 4 Kohlenstoffatomen, für gegebenenfalls durch Halogen substituiertes Alkylthio mit 1 bis 4 Kohlenstoffatomen, für Alkylcarbonyl mit 1 bis 4 Kohlenstoffatomen in der Alkylgruppe, Nitro, Fluor, Chlor, Brom und Iod steht und

n für die Zahlen 1, 2 oder 3 steht, dadurch gekennzeichnet, dass man Oxirane der Formel

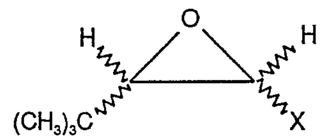

(II)

in welcher

X für Chlor oder Brom steht,

entweder

a) mit Phenolen der Formel

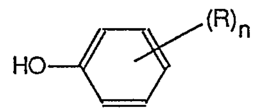

(III)

in welcher

R und n die oben angegebene Bedeutung haben, in Gegenwart einer Base sowie gegebenenfalls in Gegenwart eines Verdünnungsmittels bei Temperaturen zwischen −10 °C und+100 °C unsetzt und die dabei anfallenden Oxiran-Derivate der Formel

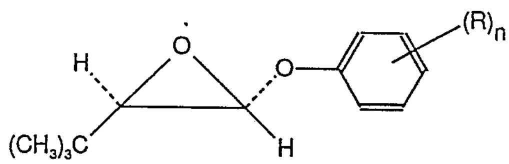

(IV)

in welcher

R und n die oben angegebene Bedeutung haben, in einem zweiten Reaktionsschritt mit 1,2,4-Triazol in Gegenwart einer Base, oder dem Natrium- oder Kalium-Salz des 1,2,4-Triazols, gegebenenfalls in Gegenwart eines Verdünnungsmittels bei Temperaturen zwischen 50 °C und 120 °C zu threo-Isomeren der Formel

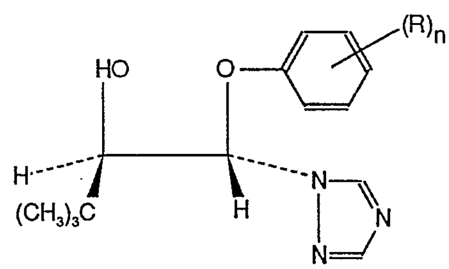

(IA)

in welcher

R und n die oben angegebene Bedeutung haben, umsetzt,

oder

b) mit 1,2,4-Triazol in Gegenwart einer Base, oder dem Natrium- oder Kalium-Salz des 1,2,4-Triazols, gegebenenfalls in Gegenwart eiens Verdünnungsmittels bei Temperaturen zwischen 0 °C und 60 °C umsetzt und das dabei anfallende Oxiran-Derivat der Formel

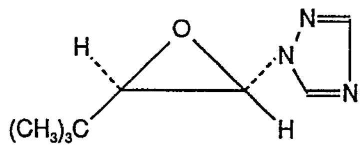

(V)

in einem zweiten Reaktionsschritt mit Phenolen der Formel

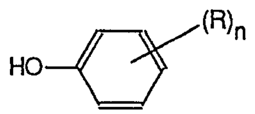

(III)

in welcher
R und n die oben angegebene Bedeutung haben, in Gegenwart eines Verdünnungsmittels bei Temperaturen zwischen 50°C und 120°C zu erythro-Isomeren der Formel

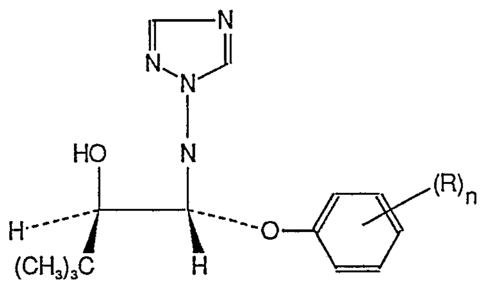

(IB)

in welcher
R und n die oben angegebene Bedeutung haben, umsetzt.

2. Verfahren gemäss Anspruch 1, dadurch gekennzeichnet, dass man 4-Chlorphenol als Ausgangsstoff der Formel (III) einsetzt.

3. Verfahren gemäss Anspruch 1, dadurch gekennzeichnet, dass man 4-Phenylphenol als Ausgangsstoff der Formel (III) einsetzt.

4. Verfahren gemäss Anspruch 1, dadurch gekennzeichnet, dass man 2-Brom-3-tert.-butyl-oxiran der Formel

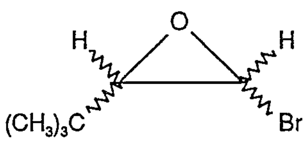

(II–2)

mit 4-Chlorphenol der Formel

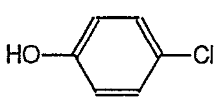

(III–1)

in Form des Natriumsalzes in Gegenwart von Acetonitril umsetzt und das dabei anfallende Derivat der Formel

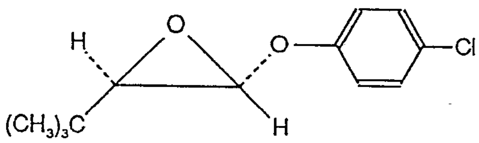

(IV–1)

in einem zweiten Reaktionsschritt mit 1,2,4-Triazol in Form des Natriumsalzes in Gegenwart von Methanol zu dem threo-1-(4-Chlorphenoxy)-3,3-dimethyl-1-(1,2,4-triazol-1-yl)-butanol-2 der Formel

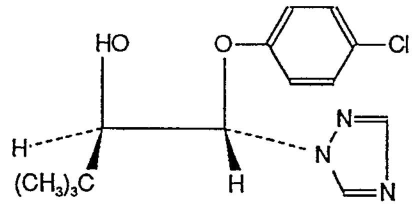

(IA–1)

umsetzt.

5. Verfahren gemäss Anspruch 1, dadurch gekennzeichnet, dass man 2-Brom-3-tert.-butyl-oxiran der Formel

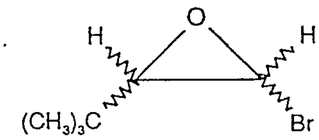

(II–2)

mit 4-Phenylphenol der Formel

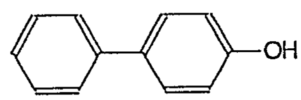

(III–2)

in Form des Natriumsalzes in Gegenwart con Acetonitril umsetzt und das dabei anfallende Oxiran-Derivat der Formel

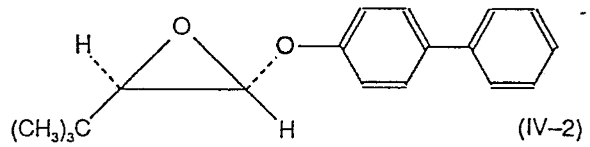

(IV–2)

in einem zweiten Reaktionsschritt mit 1,2,4-Triazol in Form des Natriumsalzes in Gegenwart von Methanol zu dem threo-1-(4-Phenylphenoxy)-3,3-dimethyl-1-(1,2,4-triazol-1-yl)-butanol-2 der Formel

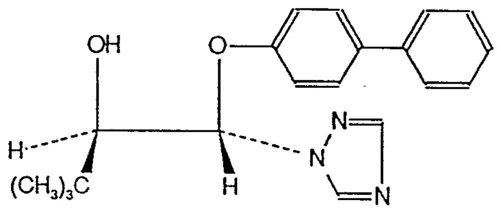

(IA–2)

umsetzt.

6. Verfahren gemäss Anspruch 1, dadurch gekennzeichnet, dass man 2-Brom-3-tert.-butyl-oxiran der Formel

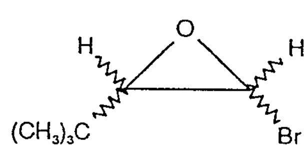

(II–2)

mit 1,2,4-Triazol in Form des Natriumsalzes in Gegenwart von Acetonitril umsetzt und das dabei anfallende Oxiran-Derivat der Formel

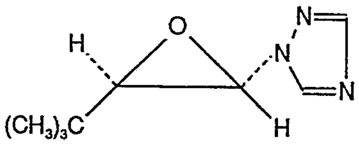

(V)

in einem zweiten Reaktionsschritt mit 4-Chlorphenol der Formel

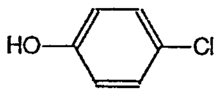

(III–1)

in Form des Natriumsalzes in Gegenwart von Methanol zu dem erythro-1-(4-Chlorphenoxy)-3,3-dimethyl-1-(1,2,4-triazol-1-yl)-butanol-2 der Formel

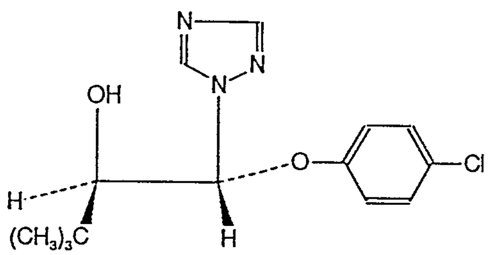

(IB–1)

umsetzt.

7. Oxiran-Derivate der Formel

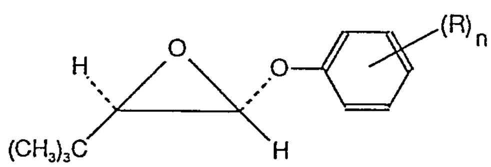

(IV)

in welcher

R für gegebenenfalls durch Halogen, Alkyl mit 1 bis 4 Kohlenstoffatomen und/oder Halogenalkyl mit 1 oder 2 Kohlenstoffatomen und 1 bis 5 Halogenatomen substituiertes Phenyl, für gegebenenfalls durch Halogen, Alkyl mit 1 bis 4 Kohlenstoffatomen und/oder Halogenalkyl mit 1 oder 2 Kohlenstoffatomen und 1 bis 5 Halogenatomen substituiertes Phenoxy, für gegebenenfalls durch Halogen substituiertes Alkyl mit 1 bis 4 Kohlenstoffatomen, für gegebenenfalls durch Halogen substituiertes Alkoxy mit 1 bis 4 Kohlenstoffatomen, für gegebenenfalls durch Halogen substituiertes Alkylthio mit 1 bis 4 Kohlenstoffatomen, für Alkylcarbonyl mit 1 bis 4 Kohlenstoffatomen in der Alkylgruppe, Nitro, Fluor, Chlor, Brom und Iod steht und

n für die Zahlen 1, 2 oder 3 steht.

8. Oxiran-Derivat der Formel

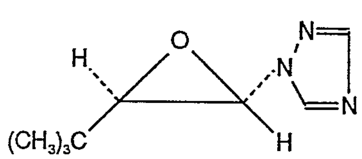

(V)

## Claims

1. Process for the synthesis of the diastereomeric forms (A) and (B) of triazolyl-O,n-acetals of the formula

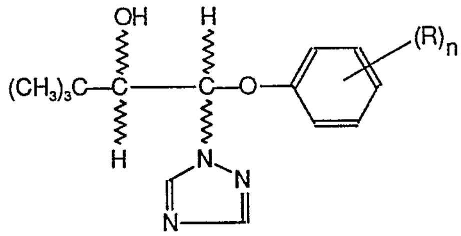

(I)

in which

R represents phenyl, which is optionally substituted by halogen, alkyl having 1 to 4 carbon atoms and/or halogenoalkyl having 1 or 2 carbon atoms and 1 to 5 halogen atoms, phenoxy, which is optionally substituted by halogen, alkyl having 1 to 4 carbon atoms and/or halogenoalkyl having 1 or 2 carbon atoms and 1 to 5 halogen atoms, alkyl having 1 to 4 carbon atoms, which is optionally substituted by halogen, alkoxy having 1 to 4 carbon atoms, which is optionally substituted by halogen, alkylthio having 1 to 4 carbon atoms, which is optionally substituted by halogen, or alkylcarbonyl having 1 to 4 carbon atoms in the alkyl group, nitro, fluorine, chlorine, bromine and iodine, and

n represents the integers, 1, 2 or 3, characterized in that oxiranes of the formula

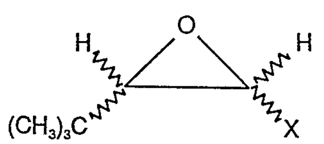

(II)

in which

X represents chlorine or bromine, are either

a) reacted with phenols of the formula

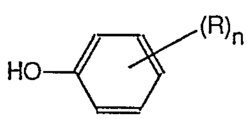

(III)

in which

R and n have the abovementioned meaning, in the presence of a base and if appropriate in the presence of a diluent at temperatures between −10°C and +100°C and, in a second reaction step, the oxirane derivatives of the formula

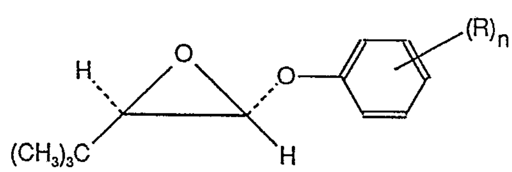

(IV)

which are thus obtained, in which

R and n have the abovementioned meaning, are

reacted with 1,2,4-triazole in the presence of a base, or with the sodium or potassium salt of 1,2,4-triazole, if appropriate in the presence of a diluent at temperatures between 50°C and 120°C to give threo-isomers of the formula

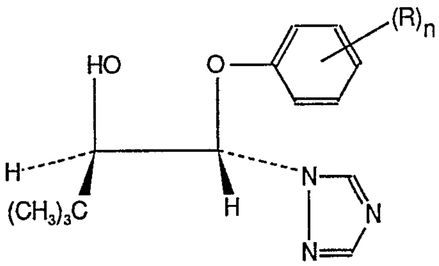

(IA)

in which
R and n have the abovementioned meaning, or
b) reacted with 1,2,4-triazole in the presence of a base, or with the sodium or potassium salt of 1,2,4-triazole, if appropriate in the presence of a diluent at temperatures between 0°C and 60°C and, in a second reaction step, the oxirane derivative of the formula

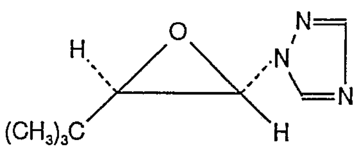

(V)

which is thus obtained, is reacted with phenols of the formula

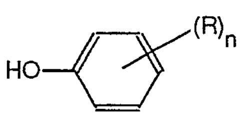

(III)

in which
R and n have the abovementioned meaning, in the presence of a base and if appropriate in the presence of a diluent at temperatures between 50°C and 120°C to give erythro isomers of the formula

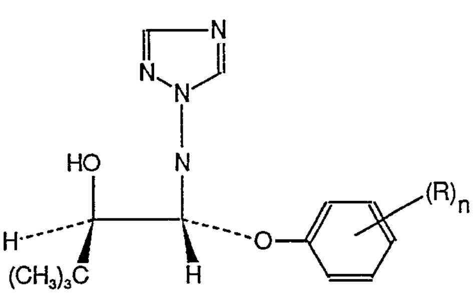

(IB)

in which
R and n have the abovementioned meaning.

2. Process according to Claim 1, characterized in that 4-chlorophenol is used as the starting material of the formula (III).

3. Process according to Claim 1, characterized in that 4-phenylphenol is used as the starting material of the formula (III).

4. Process according to Claim 1, characterized in that 2-bromo-3-tert.-butyl-oxirane of the formula

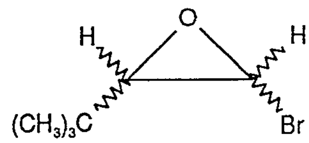

(II–2)

is reacted with 4-chlorophenol of the formula

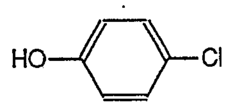

(III–1)

in the form of the sodium salt in the presence of acetonitrile and, in a second reaction step, the derivative of the formula

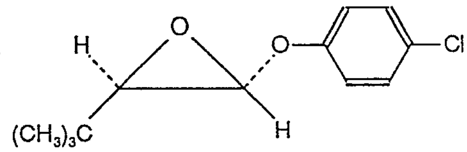

(IV–1)

which is thus obtained, is reacted with 1,2,4-triazole in the form of the sodium salt in the presence of methanol to give the threo-1-(4-chlorophenoxy)-3,3-dimethyl-1-(1,2,4-triazol-1-yl)-butan-2-ol of the formula

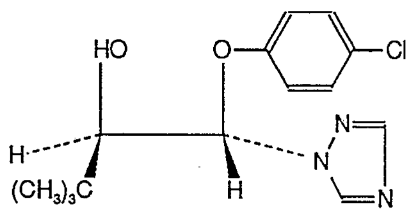

(IA–1)

5. Process according to Claim 1, characterized in that 2-bromo-3-tert.-butyl-oxirane of the formula

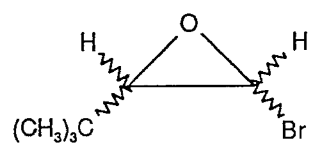

(II–2)

is reacted with 4-phenolphenol of the formula

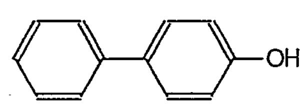

(III–2)

in the form of the sodium salt in the presence of acetonitrile and, in a second reaction step, the oxirane derivative of the formula

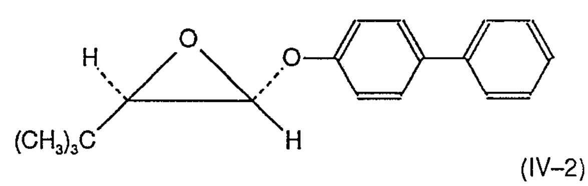

(IV–2)

which is thus obtained, is reacted with 1,2,4-triazole in the form of the sodium salt in the presence of methanol to give the threo-1-(4-phenylphenoxy)-3,3-dimethyl-1-(1,2,4-triazol-1-yl)-butan-2-ol of the formula

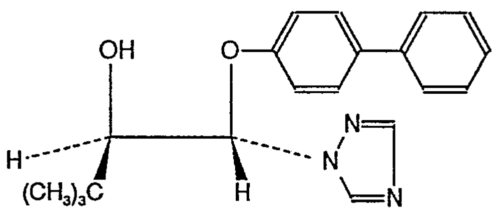
(IA–2)

6. Process according to Claim 1, characterized in that 2-bromo-3-tert.-butyl-oxirane of the formula

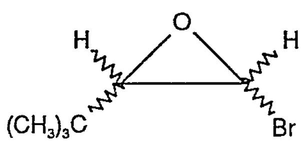
(II–2)

is reacted with 1,2,4-triazole in the form of the sodium salt in the presence of acetonitrile and, in a second step, the oxirane derivative of the formula

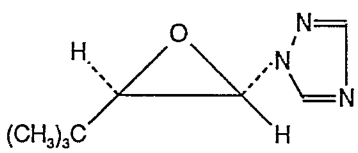
(V)

which is thus obtained, is reacted with 4-chlorophenol of the formula

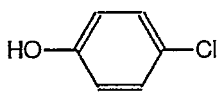
(III–1)

in the form of the sodium salt in the presence of methanol to give the erythro-1-(4-chlorophenoxy)-3,3-dimethyl-1-(1,2,4-triazol-1-yl)-butan-2-ol of the formula

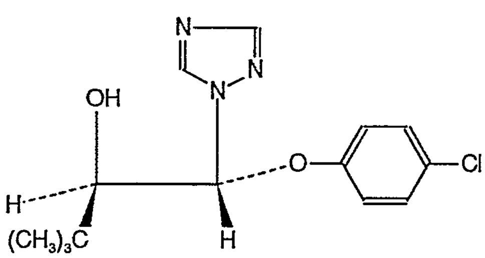
(IB–1)

7. Oxirane derivatives of the formula

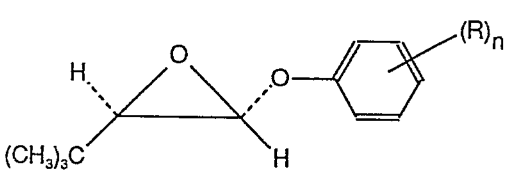
(IV)

in which
R represents phenyl, which is optionally substituted by halogen, alkyl having 1 to 4 carbon atoms and/or halogenoalkyl having 1 or 2 carbon atoms and 1 to 5 halogen atoms, phenoxy, which is optionally substituted by halogen, alkyl having 1 to 4 carbon atoms and/or halogenoalkyl having 1 or 2 carbon atoms and 1 to 5 halogen atoms, alkyl having 1 to 4 carbon atoms, which is optionally substituted by halogen, alkoxy having 1 to 4 carbon atoms, which is optionally substituted by halogen, alkylthio having 1 to 4 carbon atoms, which is optionally substituted by halogen, or alkylcarbonyl having 1 to 4 carbon atoms in the alkyl group, nitro, fluorine, chlorine, bromine and iodine, and
n represents the integers, 1, 2 or 3.

8. Oxirane derivative of the formula

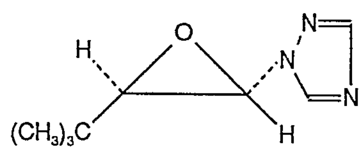
(V)

**Revendications**

1. Procédé de synthèse de formes diastéréoisomères (A) et (B) de triazolyl-O,N-acétals de formule

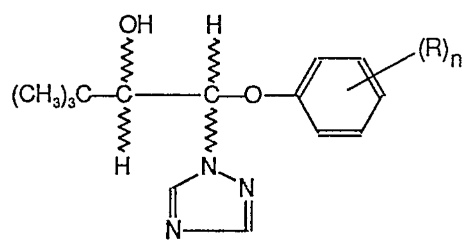
(I)

dans laquelle
R désigne un groupe phényle éventuellement substitué par un halogène, un radical alkyle ayant 1 à 4 atomes de carbone et/ou un radical halogénalkyle ayant 1 ou 2 atomes de carbone et 1 à 5 atomes d'halogènes, un groupe phénoxy éventuellement substitué par un halogène, un radical alkyle ayant 1 à 4 atomes de carbone et/ou un radical halogénalkyle ayant 1 ou 2 atomes de carbone et 1 à 5 atomes d'halogènes, un groupe alkyle de 1 à 4 atomes de carbone éventuellement substitué par un halogène, un groupe alkoxy de 1 à 4 atomes de carbone éventuellement substitué par un halogène, un groupe alkylthio de 1 à 4 atomes de carbone éventuellement substitué par un halogène, un groupe alkylcarbonyle ayant 1 à 4 atomes de carbone dans le groupe alkyle, un groupe nitro, le fluor, le chlore, le brome et l'iode et
n représente les nombres 1, 2 ou 3,
caractérisé en ce qu'on fait réagir des oxirannes de formule

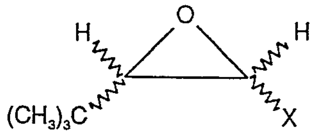

(II)

dans laquelle
X représente le chlore ou le brome, ou bien
a) avec des phénols de formule

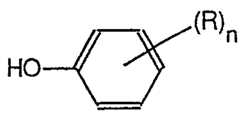

(III)

dans laquelle
R et n ont la définition indiquée ci-dessus, en présence d'une base ainsi que le cas échéant en présence d'un diluant à des températures comprises entre −10 °C et +100 °C, et on fait réagir les dérivés d'oxirannes ainsi obtenus de formule

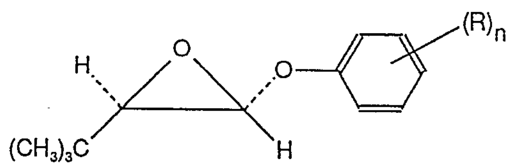

(IV)

dans laquelle
R et n ont la définition indiquée ci-dessus, dans une seconde étape de réaction avec le 1,2,4-triazole en présence d'une base, ou avec le sel de sodium ou de potassium du 1,2,4-triazole, éventuellement en présence d'un diluant, à des températures comprises entre 50 °C et 120 °C pour former des isomères thréo de formule

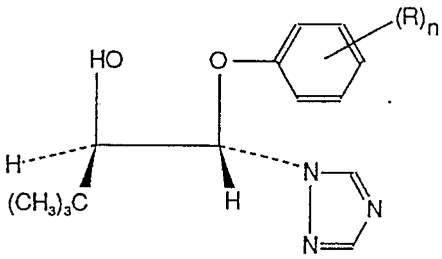

(IA)

dans laquelle
R et n ont la définition indiquée ci-dessus, ou bien
b) avec le 1,2,4-triazole en présence d'une base, ou avec le sel de sodium ou de potassium du 1,2,4-triazole, éventuellement en présence d'un diluant, à des températures comprises entre 0 °C et 60 °C, et on fait réagir le dérivé d'oxiranne ainsi obtenu de formule

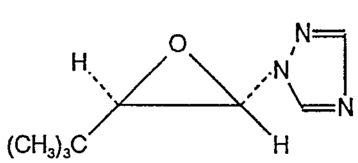

(V)

dans une seconde étape réactionelle avec des phénols de formule

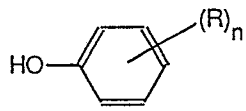

(III)

dans laquelle
R et n on la définition indiquée ci-dessus, en présence d'une base ainsi que, le cas échéant, en présence d'un diluant à des températures comprises entre 50 °C et 120 °C pour former l'isomère érythro de formule

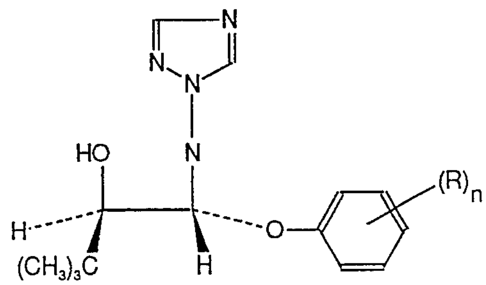

(IB)

dans laquelle
R et n ont la définition indiquée ci-dessus.

2. Procédé suivant la revendication 1, caractérisé en ce qu'on utilise le 4-chlorophénol comme composé de départ de formule (III).

3. Procédé suivant la revendication 1, caractérisé en ce qu'on utilise le 4- phénylphénol comme composé de départ de formule (III).

4. Procédé suivant la revendication 1, caractérisé en ce qu'on fait réagir le 2-bromo-3-tertiobutyloxiranne de formule

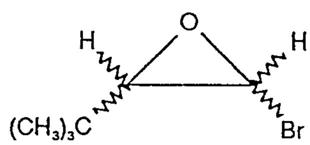

(II–2)

avec le 4-chlorophénol de formule

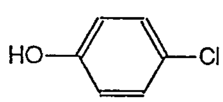

(III–1)

sous la forme du sel de sodium en présence d'acétonitrile, et on fait réagir le dérivé ainsi obtenu de formule

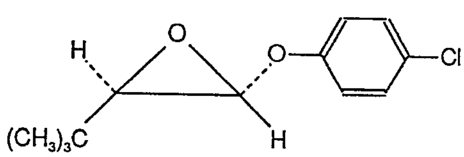

(IV–1)

dans une seconde étape réactionnelle avec le 1,2,4-triazole sous la forme du sel de sodium en présence de méthanol pour former le thréo-1-(4-chlorophénoxy)-3,3-diméthyl-1-(1,2,4-triazole-1-yl)-butanol-2 de formule

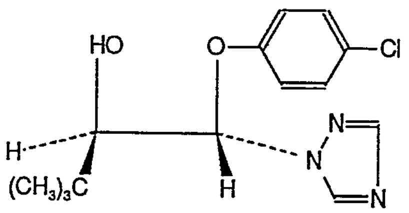

(IA-1)

5. Procédé suivant la revendication 1, caractérisé en ce qu'on fait réagir le 2-bromo-3-tertio-butyl-oxiranne de formule

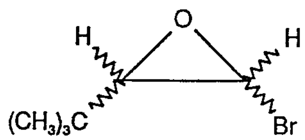

(II-2)

avec le 4-phénylphénol de formule

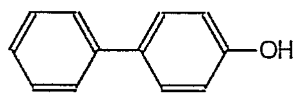

(III-2)

sous la forme de sel de sodium en présence d'acétonitrile, et on fait réagir le dérivé d'oxiranne ainsi obtenu de formule

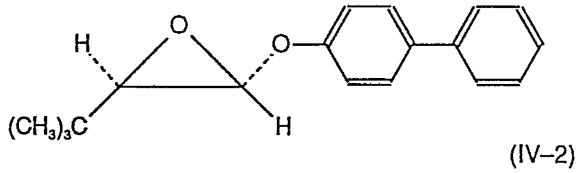

(IV-2)

dans une seconde étape réactionnelle avec le 1,2,4-triazole sous la forme du sel de sodium en présence de méthanol pour former le thréo-1-(4-phénylphénoxy)-3,3-diméthyl-1-(1,2,4-triazole-1-yl)-butanyol-2 de formule

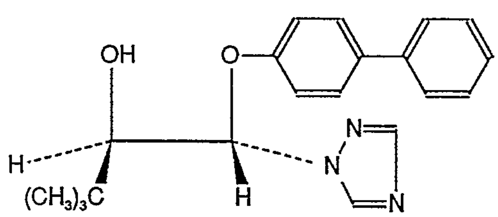

(IA-2)

6. Procédé suivant la revendication 1, caractérisé en ce qu'on fait réagir le 2-bromo-3-tertio-butyl-oxiranne de formule

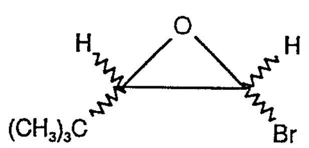

(II-2)

avec le 1,2,4-triazole sous la forme du sel de sodium en présence d'acétonitrile, et on fait réagir le dérivé d'oxiranne ainsi obtenu de formule

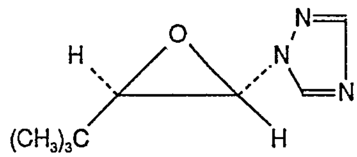

(V)

dans une seconde étape réactionelle avec le 4-chlorophénol de formule

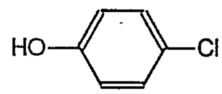

(III-1)

sous la forme du sel de sodium en présence de méthanol pour former l'érythro-1-(4-chlorophén oxy)-3,3-diméthyl-1-(1,2,4-triazole-1-yl)-butanol-2 de formule

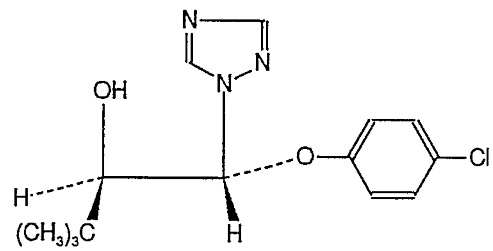

(IB-1)

7. Dérivés d'oxiranne de formule

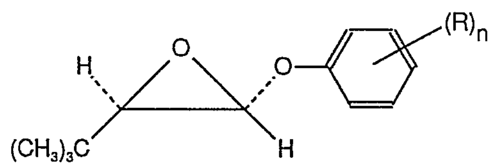

(IV)

dans laquelle
R est un groupe phényle éventuellement substitué par un halogène, un radical alkyle ayant 1 à 4 atomes de carbone et/ou un radical halogénalkyle ayant 1 ou 2 atomes de carbone et 1 à 5 atomes d'halogènes, un groupe phénoxy éventuellement substitué par un halogène, un radical alkyle ayant 1 à 4 atomes de carbone et/ou un radical halogénalkyle ayant 1 ou 2 atomes de carbone et 1 à 5 atomes d'halogènes, un groupe alkyle de 1 à 4 atomes de carbone éventuellements substitué par un halogène, un groupe alkoxy de 1 à 4 atomes de carbone éventuellement substitué par un halogène, un groupe alkylthio de 1 à 4 atomes de carbone éventuellement substitué par un halogène, un groupe alkylcarbonyl ayant 1 à 4 atomes de carbone dans le groupe alkyle, un groupe nitro, le fluor, le chlore, le brome et l'iode et représente les nombres 1, 2 ou 3.

8. Le dérivé d'oxiranne de formule

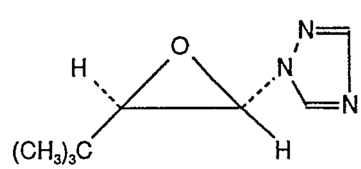

(V)